## Europäisches Patentamt

## European Patent Office

## Office européen des brevets

(11) Veröffentlichungsnummer: **0 065 747**
**B1**

# (12) EUROPÄISCHE PATENTSCHRIFT

(45) Veröffentlichungstag der Patentschrift:
15.10.86

(51) Int. Cl.⁴: **A 61 K 37/02**

(21) Anmeldenummer: **82104367.6**

(22) Anmeldetag: **18.05.82**

(54) **Nonapeptid zur Behandlung von Suchtmittelentzugserscheinungen.**

(30) Priorität: **21.05.81 CH 3306/81**

(43) Veröffentlichungstag der Anmeldung:
**01.12.82 Patentblatt 82/48**

(45) Bekanntmachung des Hinweises auf die Patenterteilung:
**15.10.86 Patentblatt 86/42**

(84) Benannte Vertragsstaaten:
**BE CH FR GB LI LU NL SE**

(56) Entgegenhaltungen:
**DE-A-2 828 433**

(73) Patentinhaber: **F. HOFFMANN- LA ROCHE & CO.
Aktiengesellschaft, CH- 4002 Basel (CH)**

(72) Erfinder: **Scherschlicht, Richard Raimund, Dr.,
Riehenstrasse 68B, D-7851 Inzlingen (DE)**
Erfinder: **Tissot, René, Dr. Prof. Clinique
Psychiatrique, de l'Université de Genève Bel Air,
CH- 1225 Chêne- Bourg (CH)**

(74) Vertreter: **Lederer, Franz, Dr., Vanderwerth,
Lederer & Riederer Patentanwälte Lucile- Grahn-
Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Das Nonapeptid Trp-Ala-Gly-Gly-Asp-Ala-Ser-Gly-Glu ist eine unter der Abkürzung DSIP (Delta-Schlaf-induzierendes Peptid) bekannte Verbindung (siehe z.B. Monnier et al., Ekperimentia 33, 548-552 (1977) oder Schoenenberger et al., Proc. Natl. Acad. Sci. USA 74, 1282-1286 [1977]) mit schlafinduzierender Wirkung.

Es wurde nun gefunden, dass diese Verbindung Eigenschaften besitzt, die sie zur Bekämpfung von Suchtmittelentzugszuständen geeignet macht.

So reagiert zum Beispiel der opiatabhängige Organismus gleich welcher Spezies auf den plötzlichen Entzug des Opiats, beispielsweise Morphins, mit einem Entzugssyndrom. Im Verlauf dieses Geschehens treten zentrale und periphere Symptome auf, deren hervorstechendstes bei der Maus eine Folge heftiger Sprünge (das sog. "jumping") ist. Die Anzahl der Sprünge pro Zeitintervall ist der Stärke der Entzugswirkung proportional und deren Zählung erlaubt eine quantitative Beurteilung des Geschehens.

Die vorliegende Erfindung betrifft die Verwendung des Nonapeptids DSIP zur Herstellung eines Mittels bei der Behandlung von Suchtmittelentzugszuständen

Obgleich das bevorzugte Einsatzgebiet des Nonapeptids die Behandlung von durch Verbindungen des Morphin-Typs (Opiate und die bekannten Ersatzpräparate) hervorgerufene Abhängigkeit ist, können auch Entzugserscheinungen, die durch die Abhängigkeit von Verbindungen des Barbiturat-Alkohol-Typs, des Amphetamin-Typs, des Cocain-Typs, des Cannabis-Typs oder des Halluzinogen-Typs (Einteilung gemäss WHO, 1974) bedingt sind, behandelt werden. Schliesslich eignet sich die Verbindung zur Behandlung der heutzutage immer häufigeren Polytoxikomanie.

Ausser dem Nonapeptid selber können auch dessen physiologisch verträgliche Salze, beispielsweise Alkalimetallsalze oder das Ammoniumsalz, verwendet werden.

Die neue Wirkung des Nonapeptids kann im Tierexperiment durch folgende Versuchsanordnung nachgewiesen werden, bei der die Hemmung des durch Naloxon ausgelösten Entzugsspringens der morphinabhängigen Maus gemessen wird:

Männlichen Mäusen (Gewicht ca. 22 g) wurde zur Erzeugung der Abhängigkeit eine Presstablette, die 75 mg Morphin (Base) enthielt und die sich im Verlauf der folgenden drei Tage unter stetiger Freisetzung des Morphins weitgehend auflöste, unter die Rückenheut implantiert. Am dritten Tag nach der Implantation wurde den Tieren zur Auslösung des Entzugsspringens der Morphinantagonist Naloxon in einer Dosis von 0,1 mg/kg subcutan verabreicht. Nach der Injektion wurden die Sprünge jedes einzelnen Tieres während 20 Minuten gezählt. Aus den Sprungsummen von jeweils 8 Tieren, die eine Gruppe bildeten, wurde der Gruppenmedianwert errechnet. Als Positivkontrolle erhielten die Tiere einer Gruppe 30 Minuten vor Verabreichung des Antagonisten 30 mg/kg Morphin s.c. Zwei bis vier Gruppen erhielten 2 Stunden vor der Naloxongabe 10 ml/kg physiologische Kochsalzlösung i.v. (Negativkontrolle). Die restlichen Gruppen erhielten 2 Stunden vor der Naloxongabe DSIP in verschiedener Dosierung (0.01, 0.015, 0.03, 0.06 und 0.1 mg/kg, i.v. sowie 0.03, 0.1 und 0.3 mg/kg s.c.). Aus allen Einzelwerten pro Dosis bzw. der Positiv- und Negativkontrollen wurden die Dosis- bzw. Kontrollmedianwerte errechnet (s. Tabelle).

Wie aus der Tabelle 1 hervorgeht, lag der Median der Negativkontrollen (Vorbehandlung mit NaCl-Lösung) bei 160 Sprüngen pro Tier innerhalb von 20 Minuten nach Naloxongabe. Der Median der Positivkontrollen (Vorbebandlung mit Morphin) betrug 43 Sprünge pro Tier oder 27% der Negativkontrolle. Das Nonapeptid DSIP senkte den Median der Sprungsumme pro Tier bei einer Dosierung von 0.015 mg/kg i.v. auf 126 oder 79% und bei einer Dosierung von 0.08 mg/kg i.v. auf 105 Sprünge oder 66% der Negativkontrolle. Nach 0.1 mg/kg s.c. sank der Median der Sprungsumme auf 138 (86%) und nach 0.3 mg/kg s.c. auf 86 (54%).

Toxikologische Studien wurden an je 12 männlichen und weiblichen Ratten sowie je 3 männlichen und weiblichen Hunden pro Dosis durchgeführt. Dabei wurde DSIP in physiologischer Kochsalzlösung gelöst und 4 Wochen lang täglich in dem Dosen von 0.6, 1.8 und 6 mg/kg intravenös verabreicht. Im Abstand von 2 Wochen wurden während dieser Studien der hämatologische, hämatochemische und der Urinstatus bestimmt, sowie das Elektrokardiogramm aufgezeichnet und analysiert. Zum Abschluss der Studien wurden alle Tiere schmerzlos getötet und es erfolgte die Autopsie nebst der Entnahme von Organproben für histologische Untersuchungen. Die genannten Dosen, deren höchste 50 mal grösser war als die zur Anwendung am Menschen vorgesehene, wurden sowohl von Ratten als auch von Hunden ohne jede Nebenwirkung vertragen. Auch bei denjenigen Tieren, die die höchste Dosis erhielten, lagen die hämatologischen, hämatochemischen und Urinwerte innerhalb der physiologischen Grenzwerte und es zeigten sich keine Veränderungen des Elektrokardiogramms. Die histologische Analyse von etwa 30 Organen sowie der Einstichstellen ergab nicht den geringsten Anhaltspunkt für Organschädigungen und zeigte, dess die lokale Verträglichkeit en der Einstichstelle eusgezeichnet war.

Das Ergebnis einer klinischen Studie mit 56 alkoholkranken bzw. methadon- oder heroinabhängigen Patienten ist in Tabelle 2 zusammengefasst. Von den 25 alkoholkranken Petienten befanden sich 8 bei der Einlieferung bereits im delirium tremens mittleren bis schweren Grades. Nach Manifestation der ersten subjektiven und objektiven Entzugszeichen erhielten die Patienten bis zu 4 intravenöse Infusionen von jeweils 0.0214 mg/kg DSIP täglich. Hierzu wurde die Substanz in physiologischer Kochsalzlösung gelöst und im Verlauf von 5 Minuten infundiert. Nach jeder dieser Infusionen berichteten die auf die Behandlung ansprechenden Patienten (38 von 56, siehe Tabelle 2) über eine Abschwächung der subjektiv empfundenen Entzugssymptomatik und das Klinikpersonal stellte ein Zurückgehen der objektiven Symptome, wie Tremor, Schwitzen, Salivation und Brechreiz fest. Das delirium tremens wurde in 6 von 8 Fällen durch die Behandlung beendet und trat in 14 von 17 Fällen, entgegen der klinischen Prognose vor Behandlungsbeginn, nicht ein. Unwirksam war DSIP in 4 der 25 Fälle von Alkoholabhängigkeit, in 3 der 15 Fälle von Methadonabhängigkeit und in 6 der 16 Fälle von Heroinabhängigkeit. Bei insgesamt 5 der 56 Fälle war eine definitive Aussage über die Wirksamkeit nicht möglich. Die Behandlungsdauer betrug maximal 3 Tage. Die Patienten waren bereits nach dieser kurzen Zeitspanne entweder ganz beschwerdefrei oder fühlten nur noch milde, leicht zu ertragende Symptome.

Das Nonapeptid und seine physiologisch verträglichen Salze können demzufolge in Form pharmazeutischer Präparate, die zur intravenösen und subkutanen Verabreichung geeignet sind, zwecks Behandlung von Suchtmittelentzugszuständen bzw. zu deren Vorbeugung verwendet werden. Die Präparate können die üblichen organischen und anorganischen inerten Trägermaterialien bzw. Lösungsmittel sowie weitere Hilfsstoffe (z.B. Konservierungs-, Stabilisierungs- oder Netzmittel, Salze zur Veränderung des osmotischen Druckes oder Puffersubstanzen) enthalten. Bei den Präparaten kenn es sich auch um Lyophilisate handeln, die kurz vor der Applikation in Lösung gebracht werden.

Als in der Humanmedizin zweckmässige Dosierungseinheit kann 1 mg der Wirksubstanz angesehen werden, die ein- bis mehrmels täglich über einen mehr oder weniger langen Zeitraum (beispielsweise 1 Woche) verabreicht werden kann. Die optimale Dosierung hängt aber sehr stark von individuellen Faktoren, dem Suchtmittel sowie dem Grad der Abhängigkeit ab und kann innerhalb relativ weiter Grenzen variieren. Ihre Bestimmung muss daher weitgehend dem fachkundigen Arzt überlassen bleiben.

**Tabelle 1:** Hemmung des Entzugsspringens der Maus nach Naloxon durch DSIP

| Substanz | Dosis | | | Sprünge/Tier (Median) | % d. Negativkontr. | Anzahl Tiere | Anzahl Gruppen |
|---|---|---|---|---|---|---|---|
| NaCl-Lsg. | 10 ml · kg⁻¹ | i.v. | | 160 | 100 | 176 | 22 |
| Morphin | 30 mg · kg⁻¹ | s.c. | | 43 | 27 | 88 | 11 |
| DSIP | 0.01 | " | i.v. | 149 | 93 | 24 | 3 |
| " | 0.015 | " | " | 126 | 79 | 24 | 3 |
| " | 0.03 | " | " | 105 | 66 | 40 | 5 |
| " | 0.06 | " | " | 148 | 93 | 24 | 3 |
| " | 0.1 | " | " | 161 | 100 | 32 | 4 |
| " | 0.03 | " | s.c. | 165 | 103 | 16 | 2 |
| " | 0.1 | " | " | 138 | 86 | 24 | 3 |
| " | 0.3 | " | " | 86 | 54 | 16 | 2 |

**Tabelle 2:** Abschwächung der Entzugssymptomatik unterschiedlicher Genese durch DSIP im klinischen Versuch.

| Missbrauchte Droge | Wirksamkeit von DSIP (1 mg pro Infusion) | | | Verträglichkeit von DSIP | | |
|---|---|---|---|---|---|---|
| | vorhanden | nicht vorhanden | unsicher | ausgezeichnet | gut | schlecht |
| Alkohol ohne delirium tremens | 14 | 3 | - | 15 | 2 | - |
| Alkohol mit delirium tremens | 6 | 1 | 1 | 7 | 1 | - |
| Methadon | 10 | 3 | 2 | 14 | 1 | - |
| Heroin | 8 | 6 | 2 | 12 | 2 | 2 |
| Gesamtpatientenzahl | 38 | 13 | 5 | 48 | 6 | 2 |

**Beispiel 1**

Injektionslösung enthaltend
Nonapeptid 1.0 mg
p-Chlor-m-kresol 1.0 mg
Natriumchlorid 8.9 mg
Wasser zu Injektionszwecken ad 1.0 ml
Die Herstellung erfolgt auf folgende Weise:
p-Chlor-m-kresol wird in $N_2$-begastem Wasser zu Injektionszwecken bei etwa 90°C gelöst. Zu dieser, auf Raumtemperatur abgekühlten Lösung werden des Nonepeptid und das Natriumchlorid zugegeben und unter Rühren gelöst. Die erhaltene Lösung wird mit $N_2$-begastem Wasser für Injektionszwecke auf des Endvolumen ergänzt, über ein sterilisiertes Membranfilter (Porengrösse 0,22 µm) filtriert und unter aseptischen Bedingungen in Ampullen zu 1 ml abgefüllt.

**Beispiel 2**

Es wurden Lyophilisate von Lösungen hergestellt enthaltend
Nonapeotid 0.55 mg 1.1 mg
D-Mannit (pyrogenfrei) 10.0 mg 10.0 mg
Wasser zu Injektionszwecken ad 1.0 ml
Dazu wird das Nonapeptid und der Mannit mit $N_2$-begastem Wasser zu Injektionszwecken gelöst und auf das berechnete Volumen gebracht. Die erhaltene Lösung wird durch ein steriles Membranfilter (Porendurchmesser 0,2 µm) filtriert und zu jeweils 1 ml unter aseptischen Bedingungen in sterile 5 ml-Ampullen (Durchmesser 13 mm) abgefüllt. Nach der Lyophilisation werden die Ampullen unter $N_2$ bei einem Druck von 550 mbar geschlossen.

Die injektionsfertige Lösung wird durch Auflösung obiger Lyophilisate in 2.2 ml 0.9%iger sterilen NaCl-Lösung erhalten.

**Patentansprüche**

1. Verwendung des Nonapeptids der Formel Trp-Ala-Gly-Gly-Asp-Ala-Ser-Gly-Glu oder eines seiner physiologisch verträglichen Salze (Verbindung I) zur Herstellung eines Mittels zur Behandlung von Suchtmittelentzugszuständen.

2. Verwendung einer Verbindung I gemäss Anspruch 1 zur Herstellung eines Mittels zur Behandlung von Zuständen, die durch den Entzug eines Suchtmittels vom Morphin-Typ Barbiburat-Alkohol-Typ, Amphetamin-Typ, Cocain-Typ, Cannabis-Typ oder Halluginogen-Typ hervorgerufen werden.

3. Verwendung einer Verbindung I gemäss Anspruch 1 zur Herstellung eines Mittels zur Behandlung von Zuständen, die durch den Entzug eines Opiats hervorgerufen werden.

4. Verwendung einer Verbindung I gemäss Anspruch 1 zur Herstellung eines Mittels zur Behandlung von Zuständen, die durch den Entzug von Morphin hervorgerufen werden.

5. Verwendung einer Verbindung I gemäss Anspruch 1 zur Herstellung eines Mittels zur Behandlung von Zuständen, die durch den Entzug von Heroin hervorgerufen werden.

6. Verwendung einer Verbindung I gemäss Anspruch 1 zur Herstellung eines Mittels zur Behandlung von Zuständen, die durch den Entzug von Alkohol hervorgerufen werden.

**Claims**

1. The use of the nonapeptide of the formula Trp-Ala-Gly-Gly-Asp-Ala-Ser-Gly-Glu or one of its physiologically compatible salts (compound I) for the manufacture of a medicament for the treatment of addictive drug withdrawal conditions.

2. The use of a compound I in accordance with claim 1 for the manufacture of a medicament for the treatment of conditions which are caused by the withdrawal of an addictive drug of the morphine type (barbiturate-alcohol type, amphetamine type, cocaine type, cannabis type or hallucinogen type).

3. The use of a compound I in accordance with claim 1 for the manufacture of a medicament for the treatment of conditions which are caused by the withdrawal of an opiate.

4. The use of a compound I in accordance with claim 1 for the manufacture of a medicament for the treatment of conditions which are caused by the withdrawal of morphine.

5. The use of a compound in accordance with claim 1 for the manufacture of a medicament for the treatment of conditions which are caused by the withdrawal of heroin.

6. The use of a compound in accordance with claim 1 for the manufacture of a medicament for the treatment of conditions which are caused by the withdrawal of alcohol.

**Revendications**

1. Application du nonapeptide de formule Trp-Ala-Gly-Gly-Asp-Ala-Ser-Gly-Glu ou d'un de ses sels physiologiquement acceptable (composé I) à la préparation d'un agent pour le traitement des états de désaccoutumance de stupéfiants.

2. Application d'un composé I selon la revendication 1 à la préparation d'un agent pour le traitement des états qui sont provoqués par la désaccoutumance d'un stupéfiant du type morphine, du type barbiturique-alcool, du type amphétamine, du type cocaïne, du type cannabis ou du type hallucinogène.

3. Application d'un composé I selon la revendication 1 à la préparation d'un agent pour le traitement des états qui sont provoqués par la désaccoutumance d'un opiacé.

4. Application d'un composé I selon la revendication 1 à la préparation d'un agent pour le traitement des états qui sont provoqués par la désaccoutumance de la morphine.

5. Application d'un composé selon la revendication 1 à la préparation d'un agent pour le traitement des états qui sont provoqués par la désac̃outumance de l'héroïne.

6. Application d'un composé I selon la revendication 1 à la préparation d'un agent pour le traitement des états qui sont provoqués par la désaccoutumance de l'alcool.